# EUROPEAN PATENT APPLICATION

(11) **EP 4 431 044 A1**
(43) Date of publication of application: **18.09.2024**
(21) Application number: 21965631.1
(22) Date of filing: 25.11.2021
(51) Int. Cl.: A61B 34/30, B25J 15/04

(54) **SLIDER AND SURGICAL INSTRUMENT**

(71) Applicant: RIVERFIELD Inc., 1070052 Tokyo (JP)
(72) Inventor: SHINDO, Koki, Tokyo 107-0052 (JP)
(74) Representative: adares Patent- und Rechtsanwälte Reininger & Partner GmbB
(86) International application number: PCT/JP2021/043244
(87) International publication number: WO 2023/095259

(57) **Abstract**

A slider is provided with a slider body arranged so as to be relatively movable in a linear direction with respect to a casing of a surgical instrument, and a rolling member arranged between the casing and the slider body and rotatable with respect to the casing and the slider body.

## Description

### TECHNICAL FIELD

The present disclosure relates to a slider and a surgical instrument that are used in a master-slave type surgical robot.

### BACKGROUND ART

In regard to master-slave type surgical robots, there have been demands for a technique to transmit external forces acting on robotic forceps to operators who operate the robots in isolated places in order to improve safety and shorten the time for doctors to learn the operation. The external forces to be transmitted to the operators are estimated based on information such as positions of actuators and driving forces. Hereinafter, robotic forceps that a surgical robot includes are also referred to as a "surgical instrument". Estimating external forces to be transmitted to an operator is also referred to as a "force sense estimation".

For example, as disclosed in Patent Document 1, there is a known method of driving a surgical instrument of a robot, in which a driving force generated by a driving source such as an actuator is transmitted through a slider or a wire, and a surgical instrument is driven by using the driving force that has been transmitted.

### PRIOR ART DOCUMENTS

### PATENT DOCUMENTS

Patent Document 1: Japanese Unexamined Patent Application Publication No. 2020-146194

### SUMMARY OF THE INVENTION

### PROBLEMS TO BE SOLVED BY THE INVENTION

The slider and the like are subj ected to a friction force, such as a static friction force and a kinetic friction force, between the slider and a casing surrounding the slider. As described above, in the force sense estimation, information on the position of the actuator or the driving force and the like is used to estimate an external force. Accordingly, the friction force acting on the slider and the like affects the force sense estimation. In other words, there is a concern that the friction force reduces accuracy of the force sense estimation.

In addition, when the driving force to be transmitted is less than the static friction force, the position of the actuator remains unchanged. This causes a concern that the force sense estimation becomes difficult when the surgical instrument is operated with the driving force less than the static friction force.

The present disclosure discloses examples of a slider and a surgical instrument that can reduce a loss caused by friction occurring when a driving force is transmitted, and inhibit deterioration in accuracy of force sense estimation.

### MEANS FOR SOLVING THE PROBLEMS

A slider according to an aspect of the present disclosure is provided with a slider body arranged so as to be relatively movable in a linear direction with respect to a casing of a surgical instrument, and a rolling member arranged between the casing and the slider body, the rolling member being rotatable with respect to the casing and the slider body.

A surgical instrument according to an aspect of the present disclosure is provided with a casing, a slider body arranged so as to be relatively movable in a linear direction with respect to the casing, and a rolling member arranged between the casing and the slider body, the rolling member being rotatable with respect to the casing and the slider body.

In the slider and the surgical instrument configured as above, the rolling member is arranged between the casing and the slider body. The rolling member is rotatable with respect to the casing and the slider body. When the slider body relatively moves in the linear direction with respect to the casing, the rolling member rotates between the casing and the slider body while in contact therewith. Accordingly, a friction force between the casing and the slider body is easily reduced.

In addition, since a static friction force is easily reduced, it is easier to move the slider body even if a driving force applied to the slider body is small. In other words, a position of an actuator that generates the driving force can be easily changed.

Furthermore, reduction in the friction force stabilizes a kinetic friction force at a certain value more easily, thus making it easy to stabilize the driving force during operation of the slider body, and a deterioration in accuracy of force sense estimation can be inhibited.

In the slider according to an aspect of the present disclosure, the rolling member is a member that has a cylindrical or columnar shape and that is supported on the slider body to be rotatable about a rotation axis intersecting the linear direction, and in a state in which the slider body moves in the linear direction, the rolling member is configured to roll while being in contact with the casing.

In the slider configured as above, the rolling member is supported by the slider body in a rotatable manner about the rotation axis. The rolling member having the cylindrical or columnar shape rotates while a circumferential surface of the rolling member is in contact with the surface of the casing. Accordingly, the friction force between the casing and the slider body is easily reduced.

In the slider according to an aspect of the present disclosure, the rolling member is a member that has a spherical shape and that is supported to be rotatable with respect to the slider body, and in a state in which the slider body moves in the linear direction, the rolling member is configured to roll while being in contact with the casing.

In the slider configured as above, the rolling member is rotatably supported by the slider body. The rolling member having a spherical shape rotates while its spherical surface is making contact with the casing. Accordingly, the friction force between the casing and the slider body is easily reduced.

### EFFECTS OF THE INVENTION

In the slider and the surgical instrument of the present disclosure, the rolling member is arranged between the casing and the slider body, thereby providing effects of reducing a loss caused by friction when a driving force is transmitted and of inhibiting a deterioration in accuracy of force sense estimation.

### BRIEF DESCRIPTION OF THE DRAWINGS

FIG. 1 is a perspective view illustrating an external view of a surgical instrument according to the present disclosure.
FIG. 2 is a perspective view illustrating an internal configuration of the surgical instrument of FIG. 1.
FIG. 3 is a perspective view illustrating an arrangement of a slider of the surgical instrument of FIG. 1.
FIG. 4 is an exploded perspective view illustrating a configuration of the slider of FIG. 3.
FIG. 5 is a sectional view illustrating arrangement relations between a rolling member, and a casing and a holder.
FIG. 6 is a sectional view illustrating a modified example of the rolling member.
FIG. 7 is a sectional view illustrating a further modified example of the rolling member.

### EXPLANATION OF REFERENCE NUMERALS

1...surgical instrument, 20...casing, 28...holder (casing), 40...slider, 41...slider body, 61...rolling member

### MODE FOR CARRYING OUT THE INVENTION

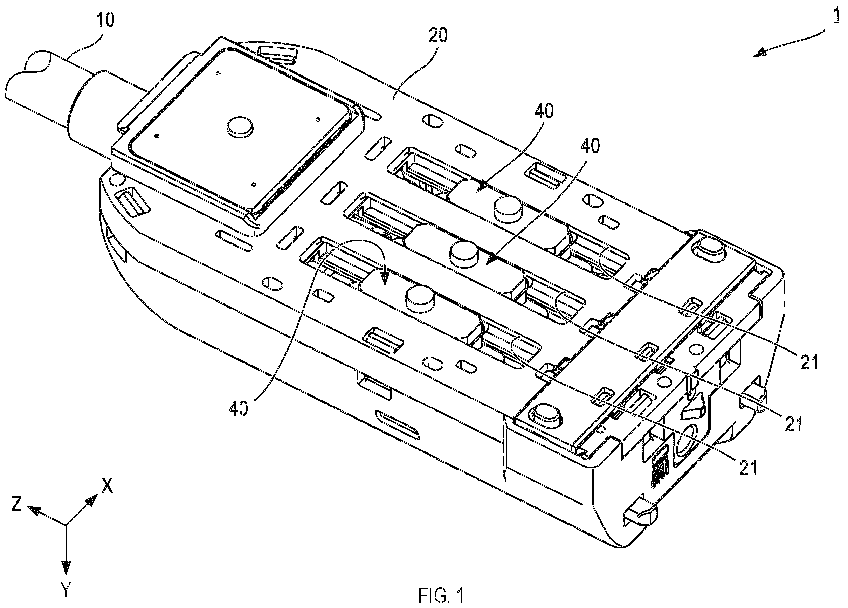

A surgical instrument according to an embodiment of the present disclosure will be described with reference to FIG. 1 through FIG. 5. A surgical instrument 1 of the present embodiment is a surgical instrument to be provided on a multi-degree-of-freedom manipulator in a surgical robot that is remotely controllable. The surgical instrument 1 is an instrument used for treatment of a patient. The surgical instrument 1 may include a configuration of, for example, forceps or the like used when endoscopic surgery is performed.

As shown in FIG. 1, the surgical instrument 1 is provided with a shaft 10 and a casing 20.

To simplify the description of the present embodiment, a direction in which the shaft 10 extends is defined as a Z-axis, and a direction toward a leading end from a base of the shaft 10 is defined as a positive direction of the Z-axis. In addition, a direction which is orthogonal to the Z-axis and along which two or more sliders 40 (described below) are aligned is defined as an X-axis, and a left direction when facing in the positive direction of the Z-axis is defined as a positive direction of the X-axis. Further, a direction orthogonal to the Z-axis and the X-axis is defined as a Y-axis, and a direction opposite to the surface where the sliders 40 are aligned in the casing 20 is defined as a positive direction of the Y-axis.

The shaft 10 is a rod-shaped member to be inserted into a body of a patient. The shaft 10 is disposed to extend from the casing 20 in the positive direction of the Z-axis. The shaft 10 is configured to have a columnar or a cylindrical shape.

For example, the shaft 10 is provided with a joint and forceps (not shown) at a leading end in the positive direction of the Z-axis.

The joint is configured to allow changes in orientations of the forceps using the driving force transmitted from the sliders 40 (described below). A specific configuration of the joint may be a general configuration that allows changes in orientations of forceps using a driving force transmitted, and is not particularly limited.

The forceps have a configuration similar to general forceps for treatment of a patient. In the present embodiment, as an example, a case in which the forceps are arranged at the leading end of the shaft 10 is described, but other instruments to be used for treatment of a patient may be arranged at the leading end of the shaft 10.

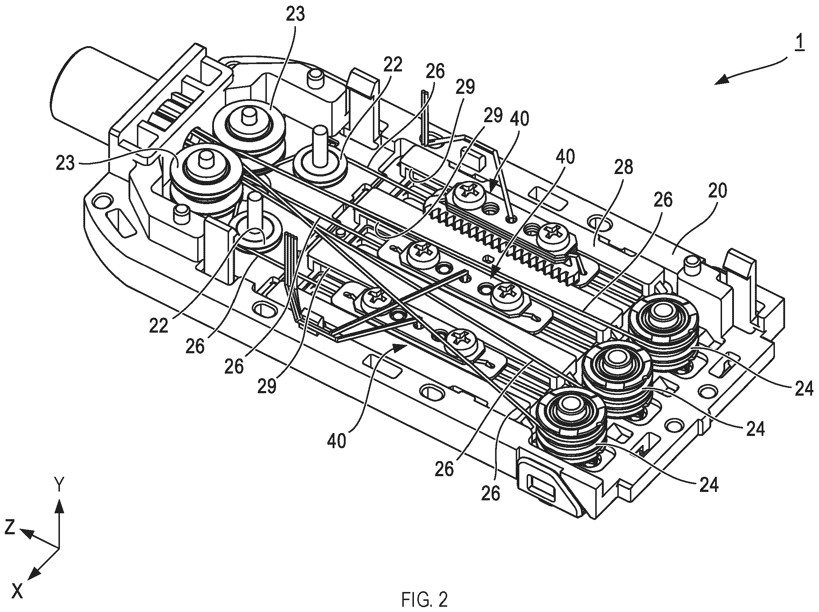

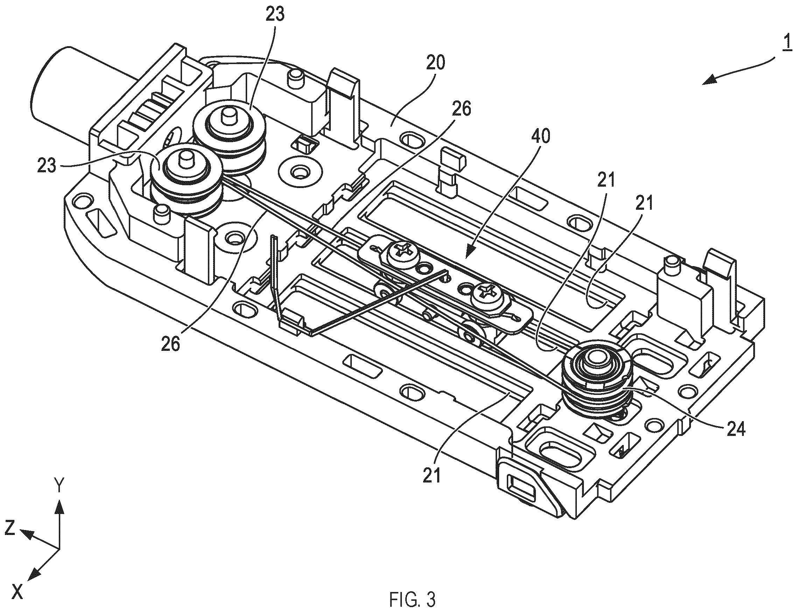

As shown in FIG. 2 and FIG. 3, the casing 20 is provided with three driven grooves 21, three sliders 40, three wires 26, and one holder 28. The holder 28 corresponds to a portion of the casing.

To simplify the description, FIG. 3 illustrates only the slider 40 and the wire 26 arranged in the driven groove 21 that is the central one in the X-axis direction. In FIG. 3, the sliders 40 and wires 26 arranged on the driven grooves 21 located at both ends in the X-axis direction are omitted.

The driven grooves 21 are elongated holes provided on an end face of the casing 20 located on a negative side in the Y-axis direction. In other words, the driven grooves 21 are elongated holes provided on an attachment/detachment face where a main body 20 and the multi-degree-of-freedom manipulator are attached. Further, the driven grooves 21 are configured to extend along the Z-axis.

The three driven grooves 21 are arranged side by side at equal intervals in the X-axis direction. The number of the driven grooves 21 may be determined based on motions of the joint, the forceps and/or the like. In other words, the number of the driven grooves 21 may be determined based on motions in accordance with a specification required for the multi-degree-of-freedom manipulator. The number of the driven grooves 21 may be more than three or less than three, in accordance with the specification required.

The sliders 40 are configured to receive the driving force from the multi-degree-of-freedom manipulator, and then transmit the driving force to the joint and/or forceps. The sliders 40 are configured to be attachable to and detachable from the multi-degree-of-freedom manipulator.

One slider 40 is arranged on each of the three driven grooves 21, one so as to be movable within the driven groove 21 in the Z-axis direction. In other words, the sliders 40 are arranged so as to be relatively movable in a linear direction with respect to the casing 20. The slider 40 may be arranged on each of all driven grooves 21, or on only a portion of the driven grooves 21.

A portion of the three sliders 40 may be configured to transmit the driving force to the forceps. The rest of the sliders 40 is configured to transmit the driving force to the joint. For example, two of the three sliders 40 are configured to transmit the driving force to the forceps. Out of the three sliders 40, one slider 40, other than the sliders 40 transmitting the driving force to the forceps, is configured to transmit the driving force to the joint.

The wires 26 are configured to transmit the driving force from the sliders 40 to the forceps or the joint. One or two wires 26 are arranged on one slider 40.

The wires 26 are wires each formed in a long shape. In the present embodiment, as an example, a case is described in which the wires 26 are made of metal materials such as stainless steel, tungsten, an alloy containing tungsten as an ingredient, and a piano wire (e.g., as specified in JIS G 3522), which are used in manipulators in surgical robot systems.

The holder 28 is a member arranged such that the three sliders 40 are arranged between the holder 28 and the casing 20. The holder 28 is provided with three slits 29 located in positions that correspond to the three driven grooves 21. The slits 29 are configured to extend along the Z-axis. The sliders 40 are arranged within the slits 29 so as to be movable in the Z-axis direction within the slits 29.

Further, as shown in FIG. 2, two first guide pulleys 22 that guide the wires 26 to the shaft 10, two second guide pulleys 23, and three third guide pulleys 24 are provided within the casing 20.

The first guide pulleys 22 are arranged closer to the shaft 10 than the sliders 40 are. The two first guide pulleys 22 are arranged side by side, one of which is on a positive side in the X-axis direction, and the other is on a negative side in the X-axis direction.

The two first guide pulleys 22 guide, to the two second guide pulleys 23, the wire 26 extending from the slider 40 located on the positive side in the X-axis direction and the wire 26 extending from the slider 40 located on the negative side in the X-axis direction.

The second guide pulleys 23 are arranged closer to the shaft 10 than the sliders 40 are, similarly to the first guide pulley 22. The two second guide pulleys 23 are arranged side by side, one of which is on the positive side in the X-axis direction, and the other is on the negative side in the X-axis direction.

The two guide pulleys 23 guide, to an inside of the shaft 10, the wire 26 extending from the first guide pulley 22 located on the positive side in the X-axis direction and the wire 26 extending from the first guide pulley 22 located on the negative side in the X-axis direction.

The third guide pulleys 24 are arranged more away from the shaft 10 than the sliders 40 are. The three third guide pulleys 24 are arranged side by side in the X-axis direction. Each of the third guide pulleys 24 is formed in a cylindrical shape and includes two or more grooves on its cylindrical surface so as to guide the corresponding wire 26.

In the present embodiment, as an example, a case is described in which three grooves are provided in different positions in the Y-axis direction on the cylindrical surfaces of each third guide pulley 24. The three third guide pulleys 24 are configured to allow the wire 26 to move from one groove that guides the wire 26 to another groove adjacent thereto.

Out of the three third guide pulleys 24, the third guide pulley 24 on the negative side in the X-axis direction causes the wire 26 extending from the slider 40 located on the negative side in the X-axis direction to, at first, move from the groove that is guiding the wire 26 to an adjacent groove, so that the wire 26 is differently positioned in the Y-axis direction and then guided to the second guide pulley 23 located on the negative side in the X-axis direction. The second guide pulley 23 on the negative side in the X-axis direction guides the wire 26 to the inside of the shaft 10.

The guide pulley 24 in the center causes the wire 26 extending from the slider 40 in the center to, at first, move from the groove that is guiding the wire 26 to an adjacent groove, so that the wire 26 is differently positioned in the Y-axis direction and then guided to the second guide pulley 23 located on the positive side in the X-axis direction. The second guide pulley 23 on the positive side in the X-axis direction guides the wire 26 to the inside of the shaft 10.

The third guide pulley 24 on the positive side in the X-axis direction causes the wire 26 extending from the slider 40 located on the positive side in the X-axis direction to, at first, move from the groove that is guiding the wire 26 to an adjacent groove, so that the wire 26 is differently positioned in the Y-axis direction and then guided to the second guide pulley 23 located on the positive side in the X-axis direction. The second guide pulley 23 on the positive side in the X-axis direction guides the wire 26 to the inside of the shaft 10.

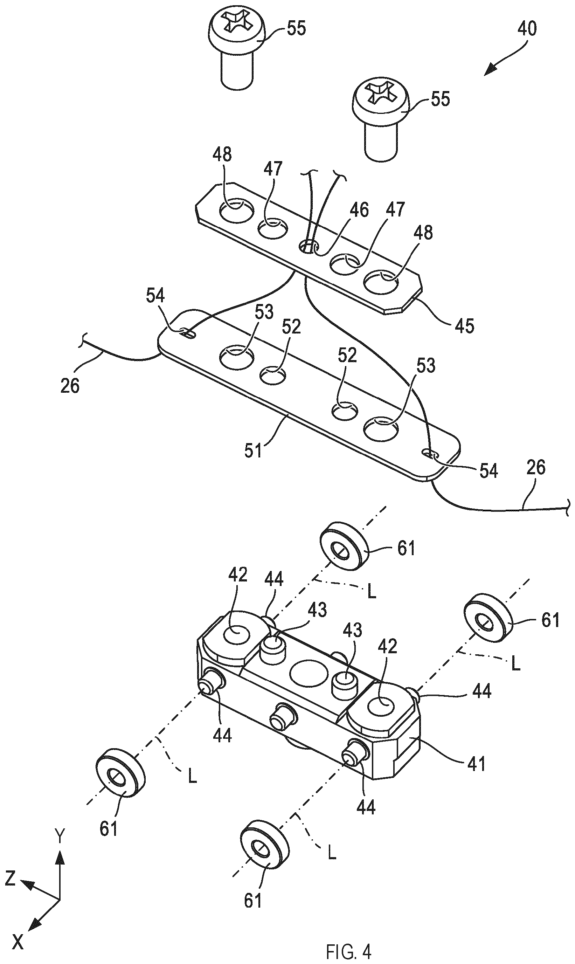

As shown in FIG. 4, the slider 40 is provided with a slider body 41, one first clamping portion 45, one second clamping portion 51, two fixing members 55, and four rolling members 61. The slider 40 may comprise an additional member.

The slider body 41 is a member formed in a pillar shape extending in the Z-axis direction. More specifically, the slider body 41 is a member formed in a square column shape. The slider body 41 is provided with two female screw holes 42, two positioning protrusions 43, and four rolling shafts 44.

The female screw holes 42 match male screws (described below) of the fixing members 55. The female screw holes 42 are screw holes used to hold the first clamping portion 45 and the second clamping portion 51. The two female screw holes 42 are screw holes formed to extend in the Y-axis direction, one of which is at an end part of the slider body 41 in the positive direction of the Z-axis, and the other of which is at an end part of the slider body 41 in the negative direction of the Z-axis.

The positioning protrusions 43 are protrusions that determine relative positions between the first clamping portion 45 and the second clamping portion 51 with respect to the slider body 41. The positioning protrusions 43 are members each having a cylindrical shape and protruding from the slider body 41 in the positive direction of the Y-axis. The two positioning protrusions 43 are provided in respective positions adjacent to the two female screw holes 42 and on a center part of the slider body 41.

The rolling shafts 44 are members that support the rolling members 61 in a rotatable manner about rotation axes L. The rolling shafts 44 are members each having a cylindrical shape and protruding from the slider body 41 in the X-axis direction. The center axis of each cylindrical shape is an axis parallel to the X-axis and is a corresponding one of the rotation axes L.

The four rolling shafts 44 are provided on the slider body 41, two of which are located, in the end part in the positive Z-axis direction, on a side face of the slider body 41 in the positive X-axis direction and a side face thereof in the negative X-axis direction, and the other two of which are located, in the end part in the negative Z-axis direction, on a side face of the slider body 41 in the positive X-axis direction and a side face thereof in the negative X-axis direction.

The first clamping portion 45 and the second clamping portion 51 are members that hold the wire 26 therebetween. The first clamping portion 45 and the second clamping portion 51 are overlapped and arranged on or above a surface of the slider body 41 located on a positive side in the Y-axis direction. The first clamping portion 45 is arranged on the positive side in the Y-axis direction, and the second clamping portion 51 is arranged on the negative side in the Y-axis direction.

The first clamping portion 45 is configured to have a rectangular plate-like shape elongated in the Z-axis direction. The first clamping portion 45 includes one insertion hole 46, two first positioning holes 47, and two first fixing holes 48.

The insertion hole 46 is a through-hole through which an end of the wire 26 is inserted, and has a smaller diameter than the first positioning holes 47 and the first fixing holes 48 do. In the present embodiment, the insertion hole 46 is located in a central area of the first clamping portion 45 in the Z-axis direction.

The first positioning holes 47 are through-holes which are each formed to have an inner diameter larger than an outer diameter of each of the positioning protrusions 43 and through which the positioning protrusions 43 are inserted. The two first positioning holes 47 are formed in respective positions on the first clamping portion 45 that are opposite to the positioning protrusions 43. In the present embodiment, the two first positioning holes 47 are located in respective positions adjacent to the insertion hole 46, in other words, an adjacent position in the positive Z-axis direction and an adjacent position in the negative Z-axis direction.

The first fixing holes 48 are through-holes which are each formed to have an inner diameter larger than an outer diameter of each male screw of the fixing members 55 and through which the fixing members 55 are inserted. The first fixing holes 48 are formed in respective positions opposite to the female screw holes 42.

The two first fixing holes 48 are formed in respective positions on the first clamping portion 45 such that the two first positioning holes 47 are arranged between the first fixing holes 48. In the present embodiment, the two first fixing holes 48 are located, one of which is adjacent in the positive Z-axis direction to the first positioning hole 47 located on a positive side in the Z-axis direction, and the other of which is adjacent in the negative Z-axis direction to the first positioning hole 47 located on the negative side in the Z-axis direction.

The second clamping portion 51 is configured to have a rectangular plate-like shape elongated in the Z-axis direction. The second clamping portion 51 is configured to have a longer dimension in the Z-axis direction than the first clamping portion 45 has. The second clamping portion 51 includes two second positioning holes 52, two second fixing holes 53, and two guide holes 54.

The second positioning holes 52 are through-holes which are each formed to have an inner diameter larger than the outer diameter of each of the positioning protrusions 43 and through which the positioning protrusions 43 are inserted. The inner diameter of each second positioning hole 52 may be the same as or different from the inner diameter of each first positioning hole 47. The two second positioning holes 52 are formed in respective positions on the second clamping portion 51 that are opposite to the positioning protrusions 43.

The second fixing holes 53 are through-holes which are each formed to have an inner diameter larger than the outer diameter of each male screw of the fixing members 55 and through which the fixing members 55 are inserted. The inner diameter of each second fixing hole 53 may be the same as or different from the inner diameter of each first fixing hole 48.

The two second fixing holes 53 are formed in respective positions opposite to the female screw holes 42. The two second fixing holes 53 are formed on the second clamping portion 51 such that the two second positioning holes 52 are located between the two second fixing holes 53.

In the present embodiment, the two second fixing holes 53 are located, one of which is adjacent in the positive Z-axis direction to the second positioning hole 52 located on the positive side in the Z-axis direction, and the other of which is adjacent in the negative Z-axis direction to the second positioning hole 52 located on the negative side in the Z-axis direction.

The guide holes 54 are through-holes through which the wire 26 is inserted and are elongated holes extending in the Z-axis direction. The two guide holes 54 are formed on the second clamping portion 51 such that the two second fixing holes 53 are located between the two guide holes 54.

In the present embodiment, the two guide holes 54 are located in respective positions, one of which is adjacent in the positive Z-axis direction to the second fixing holes 53 located on the positive side in the Z-axis direction, and the other of which is in an adjacent position in the negative Z-axis direction to the second fixing holes 53 located on the negative side in the Z-axis direction.

The positions where the two guide holes 54 are provided are contained in an area where the second clamping portion 51 protrudes more than the first clamping portion 45 in the positive Z-axis direction and an area where the second clamping portion 51 protrudes more than the first clamping portion 45 in the negative Z-axis direction, in a state in which the first clamping portion 45 and the second clamping portion 51 are attached to the slider body 41.

The fixing members 55 are configured to fix the first clamping portion 45 and the second clamping portion 51 in such a manner that the first clamping portion 45 and the second clamping portion 51 are arranged on or above the slider body 41. In addition, the fixing members 55 are configured to fix the first clamping portion 45 and the second clamping portion 51 with the wire 26 being interposed therebetween. In the present embodiment, as an example, a case in which the fixing members 55 are configured to include male screws is described.

The rolling members 61 are members that are supported by the rolling shafts 44 in a rotatable manner about the respective rotation axes L. The rolling members 61 are each formed in a cylindrical or columnar shape and configured to have a hole in a center through which the corresponding rolling shaft 44 is inserted.

The four rolling members 61 are arranged on the slider body 41, two of which are located, in the end part in the positive Z-axis direction, on the side face of the slider body 41 in the positive X-axis direction and the side face thereof in the negative X-axis direction, and the other two of which are located, in the end part in the negative Z-axis direction, on the side face of the slider body 41 in the positive X-axis direction and the side face thereof in the negative X-axis direction.

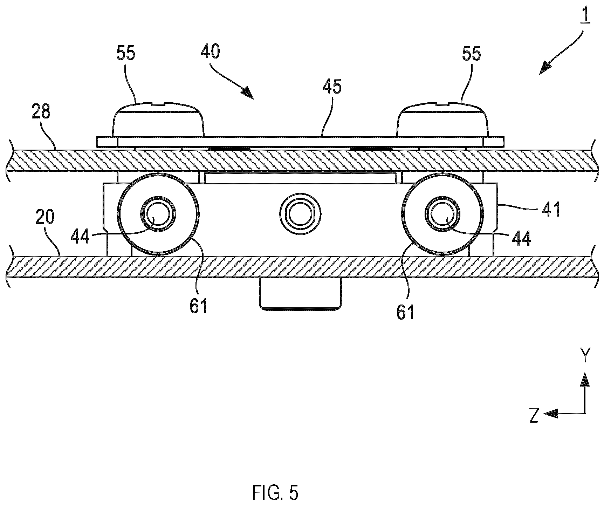

Next, movements of the sliders 40 in the surgical instrument 1 configured as above will be described with reference to FIG. 5.

The driving force to allow motion in the positive direction or negative direction of the Z-axis is transmitted from the multi-degree-of-freedom manipulator of the surgical robot to the sliders 40. The sliders 40 to which the driving forces have been transmitted linearly moves relative to the casing 20 along the driven groove 21 (see FIG. 3).

The rolling members 61 of each slider 40 have their respective circumferential surfaces in contact with a surface of the casing 20 or a surface the holder 28. When the slider 40 linearly moves relative to the casing, the rolling members 61 rotates while being in contact with the surface of the casing 20 or the holder 28.

Whether the rolling members 61 are in contact with the surface of the casing 20 or the surface of the holder 28 is determined based on an arrangement position of the slider body 41 in the Y-axis direction. The arrangement position of the slider body 41 in the Y-axis direction is determined based on the arrangement position of the wire 26. The arrangement position of the wire 26 is determined by which guide pulley, among the first guide pulleys 22, the second guide pulleys 23, and the third guide pulleys 24, guides the wire 26.

All of the four rolling members 61 arranged on one slider 40 may be in contact with the surface of the casing 20 or may be in contact with a surface of the holder 28. Alternatively, at least one of the four rolling members 61 arranged on one slider 40 may be in contact with the surface of the casing 20, and the rest of the four rolling members 61 may be in contact with the surface of the holder 28.

According to the sliders 40 and the surgical instrument 1 with the above-described configurations, the rolling members 61 are arranged between the slider body 41, and the casing 20 and the holder 28. The rolling members 61 are rotatable relative to the casing 20, the holder 28, and the slider body 41. When the slider body 41 relatively moves in a linear direction with respect to the casing 20 and the holder 28, the rolling members 61 rotate while being in contact with the casing 20 or the holder 28. Accordingly, a friction force between the casing 20 and the holder 28, and the slider body 41 is easily reduced, and thus a deterioration in accuracy of force sense estimation is easily inhibited.

In addition, since a static friction force is easily reduced, it is easier to move the slider body 41 even if the driving force applied to the slider body 41 is small. In other words, a position of an actuator that generates the driving force can be easily changed, and the surgical instrument 1 can be operated with a smaller driving force. Accordingly, the accuracy of force sense estimation is easily ensured.

The rolling members 61 are supported by the corresponding slider body 41 in a rotatable manner about the respective rotation axes L. Each rolling member 61 having a cylindrical or columnar shape rotates while a circumferential surface of the rolling member 61 is in contact with the surface of the casing 20 or the surface of the holder 28. Accordingly, the friction force between the casing 20 and the holder 28, and the slider body 41 is easily reduced.

The above-described embodiments are explained by using the example in which the rolling members 61 each have a cylindrical or columnar shape, but the rolling members 61 may have a spherical shape. The slider body 41 is provided with a configuration to allow the rolling member 61 having a spherical shape to rotate.

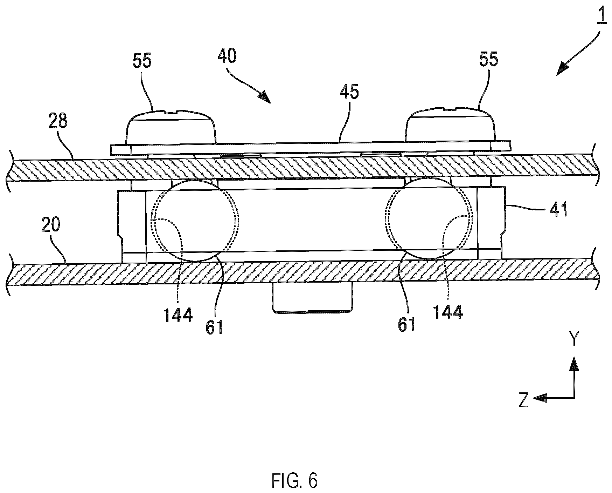

Examples of the configuration include a recessed portion and a groove, in which the rolling member 61 is arranged. As shown in FIG. 6, a holding portion 144 may be an example configuration to hold the sphere-like rolling member 61 in a rotatable manner while constantly maintaining a relative position with respect to the slider body 41.

Since the rolling member 61 having a spherical shape is rotatably supported by the slider body 41, the rolling member 61 rotates while its spherical surface making contact with the casing 20. Accordingly, the friction force between the casing 20 and the slider body 41 is easily reduced.

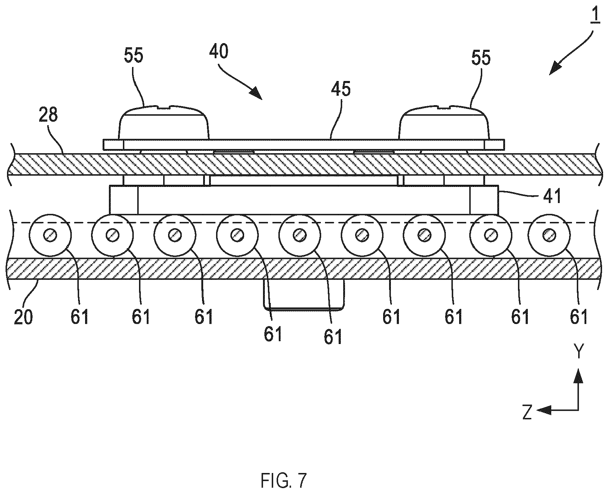

Although the above-described embodiments are explained by using the example in which the rolling member 61 is rotatably supported by the slider body 41, the rolling member 61 may be rotatably supported by the casing 20, as shown in FIG. 7.

It should be noted that the technical scope of the present disclosure is not limited to the various embodiments described above, but various changes may be made therein without departing from the spirit and scope of the present disclosure. For example, the present disclosure is not limited to one applied to the above-described embodiments, but may include an embodiment in a combination with these embodiments and is not particularly limited.

## Claims

1. A slider comprising:
a slider body arranged so as to be relatively movable in a linear direction with respect to a casing of a surgical instrument; and
a rolling member arranged between the casing and the slider body, the rolling member being rotatable with respect to the casing and the slider body.

2. The slider according to claim 1,
wherein the rolling member is a member that has a cylindrical or columnar shape and that is supported on the slider body to be rotatable about a rotation axis intersecting the linear direction, and
wherein, in a state in which the slider body moves in the linear direction, the rolling member is configured to roll while being in contact with the casing.

3. The slider according to claim 1,
wherein the rolling member is a member that has a spherical shape and that is supported to be rotatable with respect to the slider body, and
wherein in a state in which the slider body moves in the linear direction, the rolling member is configured to roll while being in contact with the casing.

4. A surgical instrument, comprising:
a casing;
a slider body arranged so as to be relatively movable in a linear direction with respect to the casing; and
a rolling member arranged between the casing and the slider body, the rolling member being rotatable with respect to the casing and the slider body.
